# EUROPÄISCHE PATENTANMELDUNG

(11) **EP 1 083 131 A2**
(43) Veröffentlichungstag der Anmeldung: **14.03.2001**
(21) Anmeldenummer: 00118726.9
(22) Anmeldetag: 30.08.2000
(51) Int. Cl.: B65D 47/34, B05B 11/00

(54) **Spender für Medien**

(30) Priorität: 08.09.1999 DE 19942792
(71) Anmelder: ING. ERICH PFEIFFER GMBH, 78315 Radolfzell (DE)
(72) Erfinder: Eberhard, Thomas, 78315 Radolfzell (DE); Breyer, Anton, 78343 Gaienhofen (DE)
(74) Vertreter: Patentanwälte Ruff, Beier und Partner

(57) **Zusammenfassung**

Um einen Pumpen-Spender (1) auf den Hals (36) einer Medienflasche (6) ohne Überlastung verkapselter Pumpenteile axial aufschnappen zu können, ist eine Sperre (20) vorgesehen, deren Sperrglied (30) den Betätigungskopf (5) gegenüber dem Befestigungsflansch (4) axial festsetzt. Dadurch kann der fertig montierte Spender (1) an der Flasche (6) befestigt werden, indem die Fügekraft in den Kopf (5) eingeleitet wird. Nach dem Ausrücken der Sperre (20) ist die Pumpe (7) über den vollen Pumphub betätigbar und ihre Betätigung kann durch Wiedereinrücken der Sperre (20) auch wieder blockiert werden.

## Beschreibung

Die Erfindung betrifft einen Spender, welcher an einem Träger, wie einem Speicher- oder Zylindergefäß, einem Griff o. dgl. mit einem Tragflansch zu befestigen ist. Der Spender kann einen volumenvariablen Medien- oder Druckraum, wie eine Pumpkammer, aufweisen, aus dem das fließfähige Medium, wie Flüssigkeit, Paste oder Creme, Pulver bzw. Gas unter Druck bis zu einem Medienauslaß gefördert werden kann, indem ein Austrag-und/oder Betätigungskopf gegenüber dem Flansch über einen Arbeitshub bewegt wird.

Bei der drehenden oder linearen bzw. axialen Befestigung des Spenders mit dem Flansch sind gerichtete Fügekräfte erforderlich, die einem Füge-Widerstand entsprechen und während der Befestigung steil ansteigen können, bis der Befestigungssitz erreicht ist. Dieser Sitz kann eine starre oder eine bewegliche Verbindung sein. Für die Betätigung des Spenders sind Laufkräfte erforderlich, welche meist in den Kopf einzuleiten sind und ebenfalls mit zunehmendem Betätigungsweg ansteigen können. Den Laufkräften entspricht ein Lauf-Widerstand, welcher z.B. durch Reibung zwischen Kolben und Zylinder, durch Federkräfte einer Rückstellfeder o. dgl., durch Betätigungskräfte für ein oder mehrere Ventile u.s.w. entsteht.

Der Lauf-Widerstand ist meist wesentlich kleiner als der Füge-Widerstand, weshalb der Kopf zweckmäßig mit der Fügekraft nicht belastet wird, um beim Fügen eine Betätigung des Kopfes bis zur Anlage an einem Anschlag bzw. bis zum Erreichen der betätigten Endlage zu vermeiden. Dieser Anschlag liegt meist verkapselt innerhalb des Spenders und kann auf eine Gegenfläche der Kolbeneinheit, wie die Endfläche einer elastischen Kolbenmanschette wirken, welche als bewegbare Begrenzung das Volumen der Medienkammer verändert. Wird diese Gegenfläche mit der Fügekraft belastet, so kann sie beschädigt bzw. undicht werden. Auch ohne Fügekräfte kann es zweckmäßig sein, trotz Einleitung der Laufkraft eine Betätigung bis zum Anschlag zu verhindern, z.B. als Originialitätssicherung, als Kindersicherung o. dgl.

Der Erfindung liegt die Aufgabe zugrunde, einen Spender zu schaffen, bei welchem Nachteile bekannter Ausbildungen bzw. der beschriebenen Art vermieden sind und der insbesondere eine Sperrung der Betätigung gegen Betätigungskräfte ermöglicht, die wesentlich höher als jeder der genannten Widerstände ist. Weiterhin soll die Fügekraft teilweise oder ausschließlich nicht über den Flansch bzw. über den Kopf einzuleiten sein. Auch soll der Spender wiederholt in den gesperrten und entsperrten Zustand zu überführen sein. Der Spender soll einfach im Aufbau und in der Betätigung sein.

Erfindungsgemäß sind Mittel vorgesehen, um wenigstens einen Teil der Fügekraft unter Umgehung des Kopfes einzuleiten, ohne daß der Kopf in der einen oder anderen Endlage steht. Dies ist zweckmäßig mit Mitteln zu erreichen, welche den Lauf-Widerstand reversibel über den Füge-Widerstand dann erhöhen, wenn die End- oder Anschlaglage oder die zugehörige, maximal mögliche, elastische Anschlagpressung noch nicht erreicht ist.

Ein Sperrglied der Sperre kann als Distanzglied zwischen Kopf und Flansch vorgesehen sein. Ist die Sperre oder deren Sperrglied nach dem Ausrücken wieder einrückbar, so kann die Sperrung der Betätigung wiederholt erfolgen. Das Sperrglied kann dabei permanent am Spender gelagert oder vom Spender vollständig trennbar sein.

Das Sperrglied kann auch gegen Aufweitung eines Spenderkörpers sichern, beispielsweise eine äußere Abschirmung oder Klammer bilden, welche den Spenderkörper gegen Radialverformung verstärkt.

Vorteilhaft ist das Sperrglied gegen Federkräfte aus- oder einrückbar, so daß es bei einfacher Betätigung sicher hält. Die zugehörigen Betätigungskräfte sind zweckmäßig quer zu den Lauf- und Fügekräften gerichtet und können zwischen diesen liegen. Zwar kann die Richtung der Lauf- und Fügekräfte voneinander abweichen, bevorzugt sind sie jedoch parallel.

Vorteilhaft deckt das Sperrglied zwischen Sperrflächen eine Vertiefung, wie einen Ringspalt, des Spenders ab, so daß kein Schmutz eindringen kann. Dadurch weist der Spender im gesperrten Zustand glattere Außenflächen als im entsperrten Zustand auf.

Das Sperrglied kann zur Befestigung des Kopfes am Betätigungsschaft der Pumpe ausgerückt oder eingerückt sein. Die zur Befestigung des Kopfes am Betätigungsschaft erforderlichen Fügekräfte sind höher als die Laufkraft und können höher oder niedriger als die Fügekräfte des Flansches sein.

Die erfindungsgemäße Ausbildung eignet sich insbesondere für Schnapp-, Bajonett- und andere Drehverschlüsse sowie zur Befestigung von Pumpen, deren Gehäuse einteilig mit oder gesondert vom Flansch ausgebildet sind. Solche Pumpen können z.B. entsprechend der deutschen Patentanmeldung 198 40 723.8 ausgebildet sein, auf deren Merkmale und Wirkungen zur Einbeziehung in die Erfindung Bezug genommen wird.

Diese und weitere Merkmale der Erfindung gehen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird. Ausführungsbeispiele der Erfindung sind in den Zeichnungen dargestellt und werden im folgenden näher erläutert. In den Zeichnungen zeigen:
- Fig. 1: einen erfindungsgemäßen Spender in teilweise geschnittener Ansicht,
- Fig. 2: den Spender gemäß Fig. 1 in Draufsicht,
- Fig. 3: das Sperrglied gemäß Fig. 1 im Diametralschnitt,
- Fig. 4: einen Axialschnitt durch Fig. 1,
- Fig. 5: das Sperrglied in Perspektive,
- Fig. 6: eine weitere Ausführung entsprechend Fig. 1,
- Fig. 7: einen Axialschnitt durch Fig. 6,
- Fig. 8: eine Draufsicht auf Fig. 6 und
- Fig. 9: eine weitere Ausführung in Perspektive.

Der Spender 1 hat zwei über einen Arbeits- oder Austraghub gegeneinander bewegliche Einheiten 2, 3 mit jeweils einem Grund- oder Spenderkörper, nämlich einem Flansch 4 und einem Kopf 5. Die Einheit 2 wird fest mit einem Träger oder Medienspeicher 6 verbunden und umfaßt das Gehäuse 8 einer Pumpe 7, wie einer Schubkolbenpumpe, deren Kolbeneinheit 9 in dem Gehäuse 8 verschiebbar ist. Die Kolbeneinheit 9 gehört zur Einheit 3 und umfaßt innerhalb des Gehäuses 8 ein Auslaßventil aus zwei gegeneinander bewegbaren Ventilteilen und eine Kolbenmanschette, die am Ende des Arbeitshubes an einer Innenschulter des Gehäuses 8 so anschlägt, daß das Auslaßventil geöffnet wird. Alle Teile liegen in einer Mittel- bzw. Tragachse 10. Das Gehäuse 8 weist einen längeren, in den Speicher 6 hineinragenden, hülsenförmigen Gehäuseteil 12 und an dessen äußeren Ende einen abgedichtet aufgeschnappten Deckel 13 auf, welcher auch einteilig mit dem Teil 12 ausgebildet sein kann. Am inneren Ende ist das Gehäuse 8 als Einlaß 11 für das Medium ausgebildet, dem in Strömungsrichtung 61 ein Einlaß- oder Kugelventil folgt, das innerhalb des Teiles 12 liegt. Der Deckel 13 hat einen radial vorstehenden, ring- oder scheibenförmigen Flansch 14, der am Träger 6 abgedichtet abzustützen oder zu verspannen ist.

Der Kopf 5 ist von einem Auslaßkanal 15 durchsetzt, der in einem Medienauslaß 16 endet und ins Freie mündet. Der Medienauslaß 16 kann eine Zerstäuberdüse, eine Strahldüse oder ein Tropfenspender sein. Er ist am freien Ende eines frei vorstehenden Stutzens 17 vorgesehen, der zur Einführung in eine Nasenöffnung geeignet ist. Statt in der Achse 10 kann der Auslaß 16 auch quer oder rechtwinklig zur Achse 10 im Mantel des Kopfes 5 liegen. Der Stutzen 17 hat einen äußersten Mantel 18 und mit Abstand darin einen inneren Mantel 19. Der Mantel 18 geht am hinteren Ende in eine ebene, ringscheibenförmige Stirnwand 21 über, deren Außenseite beiderseits des Stutzens 17 eine Handhabe 22, nämlich Druckflächen für die Finger des Benutzers bildet. Auf den Stutzen kann eine strichpunktiert angedeutete Schutzkappe aufgesetzt sein, die bis zur Handhabe 22 reicht. Die Umfangsfläche des Mantels 18 oder dieser Schutzkappe sowie die vom Auslaß 16 durchsetzte Endfläche des Stutzens 17 bzw. die Endfläche der Schutzkappe, insbesondere aber die Handhabe können die Treibfläche 22 beim Verbinden des Spenders 1 mit dem Träger 6 bilden.

Über die Innenseite der Wand 21 steht ein Mantel 23 vor, welcher eine größere Weite und eine kleinere Länge als der Mantel 18 hat. Der Deckel 13 ist von einem Kolbenschaft 24 der Einheit 9 durchsetzt, auf welchen ein Steckglied, wie eine Hülse 25 des Kopfes 5 festsitzend entgegengesetzt zur Strömungsrichtung 61, nämlich in Richtung 62 bis zum Anschlag aufgesteckt ist. Dadurch ist eine Verbindung 26 geschaffen, die den Kopf 5 in Richtung 61 durch Reibungsschluß und in Richtung 62 durch Anschlag an der Einheit 9 festsitzend hält. Der Schaft 24 ist wie der Pumpenkolben im Zentrum vom Auslaßkanal durchsetzt. Zur Befestigung des Spenders 1 am Träger 6 sowie zur Betätigung des Austraghubes wird der Kopf 5 an der Treibfläche 22 in Richtung 62 mit der Fügekraft bzw. der Betätigungskraft belastet.

Die Wand 21 bzw. Handhabe 22 ist länglich rechteckig mit konvex nach außen gekrümmten Längskanten 27 und Schmalkanten 28. Sie ist von einem in den Mantel 18 eingesetzten Einsatz 29 oder Füllstück durchsetzt, welches die Steckhülse 25 bildet. Jeder der Körper 4, 5, 6, 12, 13, 29 ist einteilig und radial bzw. axial formsteif.

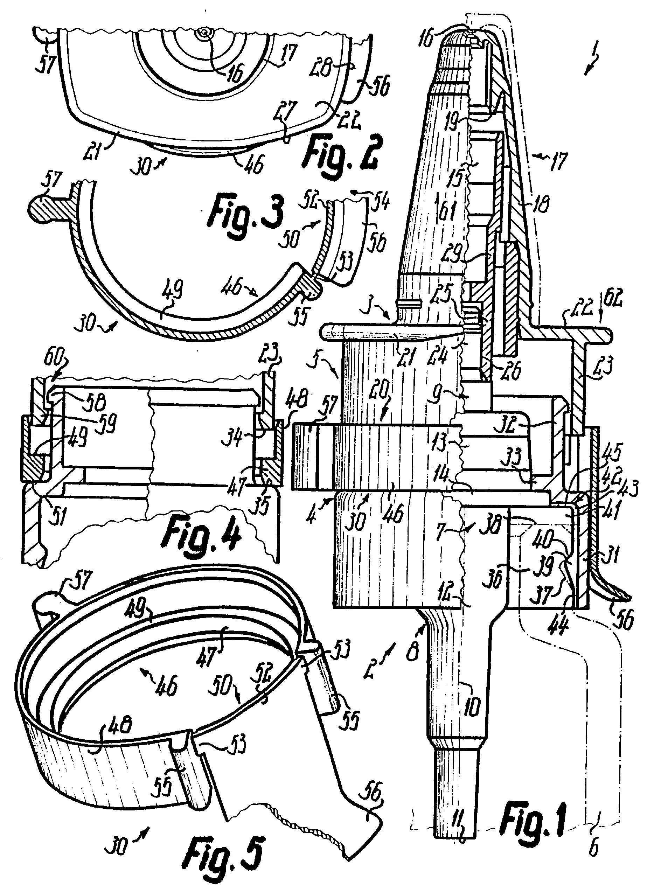

Zur Festlegung des Kopfes 5 gegenüber dem Flansch 4 in einer Stellung zwischen der Ruhelage gemäß Fig. 1 und der betätigten Endstellung ist eine Sperre 20 mit einem Sperrglied 30 vorgesehen, das als Distanzglied zwischen die Körper 4, 5 eingreift und an der Außenseite des Spenders 1 frei zugänglich liegt. Der Flansch 4 hat zwei in entgegengesetzten Richtungen 62, 61 frei ausragende Mäntel 31, 32, die über eine ringförmige Stirnwand aneinander anschließen. Vom Innenumfang des engeren Mantels 32 und im Axialabstand von dieser Stirnwand steht ein ringscheibenförmiges Flanschglied 33 vor, welches unmittelbar an der Stirnfläche des Flansches 14 drehbar anliegt und diesen gegen den Träger 6 axial spannt, so daß die Stirnwand des Flansches 4 den Flansch 14 zentrierend umgibt und der Deckel 13 vollständig innerhalb des Mantels 32 und der Stirnwand liegt. Die freie Endfläche des Mantels 23 bildet eine Schulter 34 und die Außenseite der Stirnwand zwischen den Mänteln 31, 32 bildet eine der Schulter 34 gegenüberliegende Schulter 35, zwischen die das Sperrglied 30 eingreift.

Der Träger 6 weist einen gegenüber seinem Flaschenbauch verengten Hals 36 auf, welcher am Außenumfang und mit Abstand von seiner Endfläche 38 mit einem Halte- bzw. Schnappglied 37, nämlich einer Umfangsnut, versehen ist. Am Innenumfang hat der Mantel 31 ein entsprechend vorstehendes, komplementäres Schnappglied 39. Zwei steilere, in Richtung 61 weisende Flanken der Glieder 37, 39 bilden unter Spannung aneinanderliegend die Fügung oder Verbindung 40 zwischen Spender 1 und Träger 6, nämlich einen Schnappeingriff. Die Endfläche 38 umgibt die Speicheröffnung, welche vom Gehäuse 8 mit Spaltabstand durchsetzt ist. Zur Abdichtung ist zwischen der Fläche 38 und der dieser zugekehrten Spannfläche des Flansches 14 eine Dichtung 41 eingesetzt und ebenfalls axial verspannt bzw. komprimiert. Die strichpunktiert angedeutete Dichtung 41 liegt außer am Flansch 14 und der Endfläche 38 auch dicht am Außenumfang des Gehäuseteiles 12, am Innenumfang des Mantels 31 sowie an der inneren Stirnfläche der Stirnwand des Flansches 4 mit axialer bzw. radialer Spannung an.

Der Innenraum des Speichers 6 wird belüftet, um den Druckabfall wegen Entnahme von Medium auszugleichen. Hierzu ist eine das Gehäuse 8 umgehende Belüftung 42 mit einem winkelförmigen Kanal 43 vorgesehen, der am Innenumfang des Mantels 31 den längeren, axialen Kanalast 44 und an der Innenseite der Stirnwand des Flansches 4 den kürzeren Ast 45 hat. Der Kanal 43 ist durch eine Nut im Flansch 4 gebildet, welche das bis zu den Nutflanken durchgehend ringförmige Befestigungsglied 39 durchsetzt und in deren Äste 44, 45 die Dichtung 41 radial bzw. axial so hineingedrückt ist, daß sie mit Spannung am Nutboden auch im Übergangsbereich zwischen den Ästen 44, 45 anliegt. Die für Flüssigkeiten nicht permeable aber luftdurchlässige oder semi-permeable Dichtung 41 bildet gleichzeitig ein Filter, wie ein Keimfilter, in welches die Luft vom freien Ende des Mantels 31 her durch den Ast 44 axial in Richtung 61 eintritt, wonach die Luft radial gegen die Achse 10 das Filter 41 durchströmt um schließlich entlang der Außenseite des Gehäuses 8 in Richtung 62 in den Speicher 6 zu strömen. Wegen weiterer Merkmale und Wirkungen wird zur Einbeziehung in die Erfindung auf die DE-OS 196 10 457 Bezug genommen.

Das einteilige Sperrglied 30 hat einen ring- oder teilringförmigen Sperrkörper 46, welcher sich über mindestens 180° bis 310° und höchstens über 350° um die Tragachse 10 erstreckt. Der Körper 46 hat zwei unmittelbar aneinanderschließende Mäntel 47, 48, deren äußere Umfangsflächen stetig und deren innere Umfangsflächen über eine Sperrfläche bzw. einen Anschlag 49 ineinander übergehen. Der dickere Mantel 47 bildet zwischen den Schultern 34, 35 ein Sperrglied. Dazu schlagen die Schultern 34, 35 an den voneinander abgekehrten Sperr- oder Anschlagflächen 49, 51 des Mantels 47 an. Der Mantel 48 umgibt den Mantel 23 als Abschirmung so eng, daß dieser gegen Aufweitung durch Stauchbelastung gesichert ist. Der Abstand zwischen den Sperrflächen 49, 51 entspricht etwa der Hälfte des Arbeitshubes des Kopfes 5, kann demgegenüber aber auch wesentlich größer sein. Die äußeren Umfangsflächen des Sperrgliedes 30 bzw. Sperrkörpers 46 und des Mantels 31 haben gleiche Weite.

Die von den gegeneinander gerichteten Enden des Teilringes 46 gebildete Umfangslücke ist mit einer Brücke 50 geschlossen, deren Mantel 52 dünner als der Mantel 48 sowie gegenüber diesem um seine Dicke radial nach außen versetzt ist. Der Mantel 52 ist über Bindeglieder 53, beispielsweise Sollbruch-Glieder, Gelenkglieder o. dgl. mit den einander zugekehrten Endflächen des Teilringes 46 einteilig verbunden. Diese, gegenüber dem Mantel 52 im Querschnitt geschwächten Verbindungsglieder 53 liegen nur am stromabwärtigen Ende des Mantels 52, jedoch mit diesem Ende gegenüber dem stromabwärtigen Ende des Mantels 48 zurückversetzt. Die Glieder 53 reichen über weniger als die Hälfte der axialen Länge der Glieder 46, 50 und erlauben es, die Brücke 50 um eine durch beide Glieder 53 gehende Tangentialachse gegenüber dem Sperrkörper 46 zu schwenken.

Hierzu weist die Brücke 50 eine stromaufwärtige Verlängerung des Mantels 52 als Griff 56 auf, welcher schräg vom Mantel 31 absteht und nahe bis an die freie Endfläche dieses Mantels 31 bzw. die Schulter zwischen Hals und Bauch der Flasche 6 so reicht, daß er mit einem Fingernagel untergriffen und um die Glieder 53 schwenkend nach außen gezogen werden kann. An den Griff 56 anschließend liegt der Mantel 52 am Außenumfang des Mantels 31, ggf. mit Radialspannung, an. Die Glieder 53 werden beim Schwenken entweder durch Torsionsscherung abgetrennt oder durch Trennung eines Gliedes 53 kann die Brücke 50 um das andere Glied 53 geschwenkt werden, nämlich um eine zur Achse 10 parallele Achse, so daß die Brücke 50 ggf. auch wieder in ihre Schließstellung zurückbewegt werden kann. Die Brücke 50 schließt die Öffnung oder Ringlücke 54 des Sperrkörpers 46, die als Aus- oder Einführmaul 54 dazu dient, den Sperrkörper 30 radial vom Spender 1 abzuziehen.

Hierzu bildet der Sperrkörper 46 mit seinen Enden radial federnde Schenkel, deren Abstand an der Lücke 54 wesentlich kleiner als der Durchmesser des zugehörigen Mantels 32 ist. Diese Schenkelenden gleiten beim Abziehen am Mantel 32, wobei sie zuerst federnd aufweiten und dann wieder in ihre Ausgangslage zurückkehren. An den Enden stehen vom Außenumfang des Sperrkörpers 46 stegförmige Flankenvorsprünge 55 radial vor, die nur über die gesamte Länge des Sperrkörpers 46 reichen und deren einander zugekehrte Flanken das radial nach außen divergierende Maul 54 trichterförmig begrenzen. Dadurch kann der Sperrkörper 46 auch wieder auf den Mantel 32 aufgeschoben werden, wobei seine Schenkel federnd aufweiten und dann in die Sperrlage zurückspringen. Die Brücke 50 weist die Sperrfläche 49 nicht auf. Zum Abziehen und Aufsetzen des Sperrkörpers 46 ist ein Griff 57 an dem vom Maul 54 abgekehrten Umfang des Sperrkörpers 46 vorgesehen.

Der Körper 30 und die Handhabe 22 sind symmetrisch zu einer gemeinsamen Axialebene ausgebildet, so daß die Handhabe 56 an einer Schmalseite 28 und der Griff 57 an der davon abgekehrten Schmalseite gemäß Fig. 2 radial vorstehen. Ebenfalls in Axialansicht steht der Sperrkörper 46 nicht oder wesentlich weniger als die Griffe 56, 57 über die Längsseiten 27 vor.

Das Sperrglied 30 liegt im Bereich der Glieder oder Nocken 58, 59 einer Abziehsicherung 61, durch welche das Abziehen des Körpers 5 vom Körper 4 formschlüssig verhindert ist. Der ringförmige Nocken 58 steht am freien Ende über den Mantel 32 nach außen vor. Der ringförmige Nocken 59 steht am freien Ende über den inneren Umfang des Mantels 23 vor. Bei einer Bewegung des Kopfes 5 aus der Ruhelage in Richtung 62 schlagen die Nocken 59 am Nocken 58 an. In der genannten Axialebene des Sperrgliedes 30 ist die radiale Höhe der Nocken 58, 59 reduziert oder vollständig unterbrochen. Beim Aufsetzen des Kopfes 5 in Richtung 62 laufen die Nocken 58, 59 unter Radialfederung der Mäntel 23, 32 aneinander vorbei bis sie als Schnappverbindung in ihre Funktionslage zurückspringen. Der Nocken 59 liegt teilweise oder ganz im Mantel 48.

Die Kolbeneinheit 9 benötigt axiale Laufkräfte, um den Lauf-Widerstand zu überwinden, welcher durch eine im Gehäuse 8 liegende Rückstellfeder mit zunehmendem Hubweg ansteigen kann. Um die Verbindung 26 herzustellen, bedarf es einer axialen Verbindungskraft, welche den Verbindungs-Widerstand des Preßsitzes 26 überwindet. Um die Flanschkappe 31 bzw. das Glied 39 in den genannten Fügeeingriff mit dem Speicher 6 zu bringen, bedarf es einer axialen Fügekraft, welche den Füge-Widerstand überwindet. Zur Herstellung der Fügung 40 zwischen Spender 1 und Träger 6 wird der Spender 1 mit einem Preßwerkzeug so gefaßt, daß es mit seinen Preßflächen an mindestens einer der genannten Treibflächen 22 anliegt. Dann wird der Spender 1 mit diesem Werkzeug in Richtung 62 auf den Hals 36 linear aufgeprellt, wobei zuerst der Kopf 5 den Teilhub bis zum Anschlag an der Sperrfläche 49 durchführt und danach die Fügekraft allein über den Mantel 47 auf den Flansch 4 überträgt.

Wegen des vorangegangenen Teilhubes ist die Rückstellfeder stärker vorgespannt, so daß das Gehäuse 8 bereits vor der Herstellung der Fügung 40 durch diese Feder mit dem Flansch 40 fest gegen die Endfläche 38 bzw. die Dichtung 41 gespannt wird. Danach führt der Flansch 4 gegenüber dem Gehäuse 8 noch einen Axialweg bis zur Erreichung seiner Fügeposition aus. Der Flansch 4 und das Gehäuse 8 bzw. dessen zugehöriger Teil 12 bzw. 13 können auch einteilig miteinander sein. Zur Durchführung eines Austraghubes wird das Sperrglied 30 in der beschriebenen Weise entfernt oder ausgerückt und zur darauffolgenden Sperrung des Arbeitshubes wieder eingerückt. Der Teilweg des Arbeitshubes bis zum Anschlag an der Sperrfläche 49 ist so gewählt, daß dabei das Auslaßventil der Pumpe 7 nicht öffnet. Beim Rückhub aus dieser Zwischenlage wird jedoch über den Einlaß 11 Medium aus dem Speicher 6 in die Pumpkammer angesaugt, so daß bereits mit dem nächsten oder übernächsten Pumphub Medium aus dem Auslaß 16 ausgetragen werden kann.

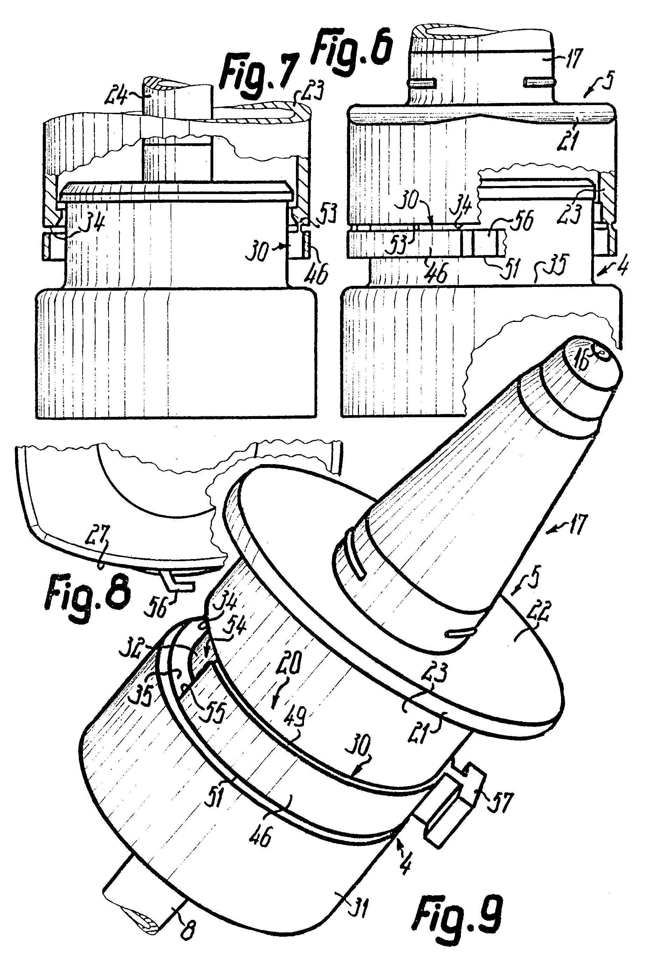

Gemäß den Figuren 6 bis 8 erstreckt sich der Sperrkörper 46 als konstant dicker, geschlossener Ringmantel ununterbrochen über den Umfang und reicht höchstens bis an den Mantel 23 bzw. bis an dessen Außenweite. Das Sperrglied 30 ist mit der Endfläche 34 über die Bindeglieder 53 verbunden, die beabstandet über den Umfang verteilt sind und allein die genannte Fügekraft auf den Ring 30 übertragen. Die Handhabe 56 steht in Axialansicht über die Längsseite 27 vor und liegt im Bereich einer Sollbruch- bzw. Schwachstelle des Ringes 30. Wird die Handhabe 56 kippend radial nach außen gezogen, so reißt diese Bruchstelle und der Ring 30 kann als offenes Band entfernt werden. Die Manteldicke des Sperrkörpers 46 ist kleiner als die des Mantels 43. Das Glied 46 liegt im axialen Spaltabstand vom Mantel 23 und dieser Spaltabstand ist nur durch die Glieder 53 überbrückt.

Gemäß Fig. 9 wird der Kopf 5 bereits in der Ruhelage blockiert, da in dieser Lage die Flächen 49, 51 an den Flächen 34, 35 spielfrei oder mit geringer Axialspannung anliegen. Für das Zusammenfügen der Körper 4, 5 kann das Sperrglied 30 mit einem dieser Körper, insbesondere gemäß den Figuren 1 und 9 mit dem Körper 4 und gemäß Fig. 6 mit dem Körper 5, eine vormontierte Einheit bilden. Außerdem ist das am Spender 1 montierte Sperrglied 30 gemäß den Figuren 1 und 9 gegenüber beiden Körpern und gemäß Fig. 6 gemeinsam mit dem Körper 5 gegenüber dem Körper 4 um die Achse 10 drehbar.

In allen Ausführungsformen sind für einander entsprechende Teile die gleichen Bezugszeichen verwendet, weshalb alle Beschreibungsteile sinngemäß für alls Ausführungsformen gelten. Jedes Merkmal jeder Ausführungsform kann bei jeder anderen Ausführungsform in Addition oder Kombination vorgesehen sein. Die gezeigten Größenverhältnisse sind bei einer Gesamtlänge des Spenders 1 von höchstens 100 oder 80 mm für viele Anwendungsbereich günstig. Die Wirkungen und Eigenschaften können genau oder nur im wesentlichen bzw. etwa wie beschrieben vorgesehen sein oder für entsprechende Anwendungen auch stärker davon abweichen. Die Körper 4, 5 können auch gegeneinander über den gesamten Hub verdrehgesichert sein, beispielsweise indem die Nocken 58, 59 oder die zugehörigen Umfangsflächen der Mäntel 23, 32 aneinander gleitende Abflachungen aufweisen, die gegenüber den zugehörigen Umfangsflächen radial nach innen versetzt sind.

## Patentansprüche

1. Spender für Medien mit Spendereinheiten (2, 3) und Spenderkörpern (4, 5), wie einem Kopf (5) zur Betätigung und einem Flansch (4) zur Befestigung an einem Träger (6) oder Medienspeicher in einer Tragachse (10), dadurch gekennzeichnet, daß der zweite Spenderkörper (5) relativ zum ersten Spenderkörper (4) gegen einen Lauf-Widerstand aus einer Ruhelage über Zwischenlagen in eine betätigte Endlage bewegbar ist sowie zur Befestigung des ersten Spenderkörpers (4) am Träger (6) gegen einen Füge-Widerstand eine Treibfläche (22) aufweist, und daß Mittel, wie eine Sperre (20), zur Befestigung des ersten Spenderkörpers (4) am Träger (6) mittels des zweiten Spenderkörpers (5) vorgesehen sind.

2. Spender nach Anspruch 1, dadurch gekennzeichnet, daß der Füge-Widerstand größer als der Lauf-Widerstand ist, daß die Befestigungsmittel (20) als Montagemittel in einer von der Endlage abweichenden Lage des zweiten Spender Körpers (5) den Lauf-Widerstand relativ zum Füge-Widerstand erhöhen, daß die Montagemittel (20) zur Entsperrung von der Außenseite des Spenders betätigbar sind und daß die Montagemittel (20) ein unter dem Füge Widerstand an mindestens einem der Spenderkörper (4, 5) mit einer Sperrfläche (49, 51) eines Sperrkörpers (46) unmittelbar anliegendes Sperrglied (30) aufweisen, daß insbesondere das Sperrglied (30) einteilig ist, und daß vorzugsweise das Sperrglied (30) frei zugänglich an der Außenseite des Spenders (1) liegt.

3. Spender nach Anspruch 1 oder 2, dadurch gekennzeichnet, daß der erste Spenderkörper (4) eine den zweiten Spenderkörper (5) beim Schub gegen den Träger (6) stützende Gegenschulter (35) aufweist, die stromaufwärts eines stromabwärtigen Endes (58) des ersten Spenderkörpers (4) liegt, daß insbesondere ein Sperrglied (30) der Befestigungsmittel (20) bzw. der zweite Spenderkörper (5) den ersten Spenderkörper (4) am Umfang umgibt, und daß vorzugsweise ein Sperrkörper (46) des Sperrgliedes (30) einen inneren Sperrvorsprung aufweist.

4. Spender nach einem der vorhergehenden Ansprüche, dadurch gekennzeichnet, daß sich ein Sperrglied (30) der Befestigungsmittel (20) über mindestens 310° bis 360° um die Tragachse (10) erstreckt und Mittel (50) gegen Aufweitung des Sperrgliedes (30) vorgesehen sind, daß insbesondere das Sperrglied (30) in einer Axialebene unterschiedliche Radialerstreckungen hat und daß vorzugsweise das Sperrglied (30) unterschiedliche Axialerstreckungen aufweist.

5. Spender nach einem der vorhergehenden Ansprüche, dadurch gekennzeichnet, daß ein Sperrglied (30) der Befestigungsmittel (20) mindestens einen radial zugänglichen Handgriff (56, 57) aufweist, daß insbesondere der Handgriff (56) einer Schulter des Trägers (6) mit geringem Abstand unmittelbar gegenüberliegt, und daß vorzugsweise zwei axial bzw. radial unterschiedlich weit vorstehende Handgriffe (56, 57) vorgesehen sind.

6. Spender nach einem der vorhergehenden Ansprüche, dadurch gekennzeichnet, daß ein Sperrglied (30) der Befestigungsmittel (20) um die Tragachse (10) über annähernd 360° ringförmig ist, daß insbesondere ein Ringausschnitt (52) von einem Sperrkörper (46) des Sperrgliedes (30) zur Bildung einer Ringlücke (54) entfernbar ist, und daß vorzugsweise der Ringausschnitt (52) einteilig mit mindestens einer Sperrfläche (49, 51) ausgebildet sowie mit dem Sperrkörper (46) über ein verformbares Bindeglied (53), wie ein Sollbruchglied, verbunden ist.

7. Spender nach einem der vorhergehenden Ansprüche, dadurch gekennzeichnet, daß den Befestigungsmitteln (20) eine Abzugsicherung (60) gegen Auseinanderziehen der Spenderkörper (4, 5) zugeordnet ist, daß insbesondere ein Sperrglied (30) der Befestigungsmittel (20) die Abzugsicherung (60) abschirmt, und daß vorzugsweise das Sperrglied (30) quer zur Tragachse (10) benachbart zum Träger (6) liegt.

8. Spender nach einem der vorhergehenden Ansprüche, dadurch gekennzeichnet, daß eine Sperrfläche (49) der Befestigungsmittel (20) im Abstand von mindestens einer Endfläche eines einteiligen Sperrgliedes (30) liegt, daß insbesondere ein Sperrglied (30) in der Ruhelage mindestens einen der Spenderkörper (4, 5) als Klammer außen abdeckt bzw. gegen Radialverformung sichert, und daß vorzugsweise ein Sperrkörper (46) der Befestigungsmittel (20) im Abstand von sowie zwischen zwei voneinander abgekehrten Endflächen die gegen die Tragachse (10) gerichtete Sperrfläche (49) aufweist.

9. Spender nach einem der vorhergehenden Ansprüche, dadurch gekennzeichnet, daß ein Sperrglied (30) der Befestigungsmittel (20) mit mindestens einem der Spenderkörper (4, 5) eine Montage-Einheit bildet, daß insbesondere das Sperrglied (30) einteilig mit mindestens einem der Spenderkörper (4, 5) ausgebildet ist, und daß vorzugsweise das Sperrglied (30) mit dem Spenderkörper (4 bzw. 5) über mindestens ein Sollbruch-Glied (53) verbunden ist.

10. Spender nach einem der vorhergehenden Ansprüche, dadurch gekennzeichnet, daß die Sperre (20) nach der Entsperrung wieder einrückbar ist, daß insbesondere ein Sperrglied (30) der Sperre (20) ein Ausführmaul (54) für mindestens einen der Spenderkörper (4, 5) hat, und daß vorzugsweise das Ausführmaul (54) von mindestens einem radial vorstehenden Flankenvorsprung (55) begrenzt ist, der eine vom Sperrglied (30) verschiedene Wandungsdicke hat.

11. Spender nach einem der vorhergehenden Ansprüche, dadurch gekennzeichnet, daß ein Sperrkörper (46) der Befestigungsmittel (20) eine Länge hat, die höchstens so groß wie seine Weite ist, daß insbesondere der Sperrkörper (46) einen gewindefreien Abschnitt des zweiten Spenderkörpers (5) mit Radialabstand umgibt, und daß vorzugsweise die Spenderkörper (4, 5) bei eingerückter Sperre (20) über einen Leerweg relativ zueinander bewegbar sind.

12. Spender nach einem der vorhergehenden Ansprüche, dadurch gekennzeichnet, daß er eine Pumpe (7), wie eine mit dem Kopf (5) zu betätigende Schubkolbenpumpe, mit zwei relativ zueinander bewegbaren Pumpen-Einheiten (8, 9), wie einem Pumpengehäuse und einer Kolbeneinheit, umfaßt, von denen eine mittels der Befestigungsmittel (20) mit dem Flansch (4) am Träger (6) zu lagern ist, daß insbesondere der Flansch (4) in einer axialen Bewegung in axial gesicherte Fügung, wie einen Schnappeingriff (40), mit dem Träger (6) zu bringen ist, und daß vorzugsweise der Flansch (4) einen Kanal (43), wie einen Belüftungskanal zur Belüftung des Trägers (6), begrenzt.
